# EUROPÄISCHE PATENTSCHRIFT

(11) **EP 2 784 512 B1**
(45) Veröffentlichungstag und Bekanntmachung des Hinweises auf die Patenterteilung: **10.05.2017**
(21) Anmeldenummer: 14161912.2
(22) Anmeldetag: 27.03.2014
(51) Int. Cl.: G01N 33/68

(54) **Verfahren zur ejakulatbezogenen Prognose der Fertilität eines Bullen**
Method for predicting the fertility of a bull based on its ejaculate
Procédé de prédiction de la fertilité d'un taureau en fonction de l'éjaculat

(30) Priorität: 28.03.2013 EP 13161581
(43) Veröffentlichungstag der Anmeldung: 01.10.2014
(73) Patentinhaber: FZMB GmbH Forschungszentrum für Medizintechnik und Biotechnologie, 99947 Bad Langensalza (DE)
(72) Erfinder: Kölle, Sabine, Dublin, 18 (IE); Miethe, Peter, 99947 Bad Langensalza (DE); Richter, Ina Gabriele, 99947 Bad Langensalza (DE)
(74) Vertreter: dompatent von Kreisler Selting Werner - Partnerschaft von Patent- und Rechtsanwälten mbB

(56) Entgegenhaltungen:
- WO-A2-2013/012749
- DATABASE BIOSIS [Online] BIOSCIENCES INFORMATION SERVICE, PHILADELPHIA, PA, US; April 2000 (2000-04), REVAH IRMA ET AL: "Physiological state of bull sperm affects fucose- and mannose-binding properties", XP055068600, Database accession no. PREV200000223750 & REVAH IRMA ET AL: "Physiological state of bull sperm affects fucose- and mannose-binding properties", BIOLOGY OF REPRODUCTION, Bd. 62, Nr. 4, April 2000 (2000-04), Seiten 1010-1015, ISSN: 0006-3363
- SS SUAREZ: "Formation of a Reservoir of Sperm in the Oviduct", REPRODUCTION IN DOMESTIC ANIMALS, Bd. 37, Nr. 3, 1. Juni 2002 (2002-06-01), Seiten 140-143, XP55125680, ISSN: 0936-6768, DOI: 10.1046/j.1439-0531.2002.00346.x
- D RATH ET AL: "Sperm Interactions from Insemination to Fertilization", REPRODUCTION IN DOMESTIC ANIMALS, Bd. 43, 22. November 2008 (2008-11-22), Seiten 2-11, XP55125681, ISSN: 0936-6768, DOI: 10.1111/j.1439-0531.2008.01250.x
- D WABERSKI: "Binding of boar spermatozoa to oviductal epithelium in vitro in relation to sperm morphology and storage time", REPRODUCTION, Bd. 131, Nr. 2, 1. Februar 2006 (2006-02-01), Seiten 311-318, XP055068531, ISSN: 1470-1626, DOI: 10.1530/rep.1.00814
- ELLINGTON JOANNA E ET AL: "Higher-quality human sperm in a sample selectively attach to oviduct (fallopian tube) epithelial cells in vitro", FERTILITY AND STERILITY, Bd. 71, Nr. 5, Mai 1999 (1999-05), Seiten 924-929, XP055068574, ISSN: 0015-0282
- WABERSKI D ET AL: "Importance of sperm-binding assays for fertility prognosis of porcine spermatozoa", THERIOGENOLOGY, LOS ALTOS, CA, US, Bd. 63, Nr. 2, 15. Januar 2005 (2005-01-15), Seiten 470-484, XP027746960, ISSN: 0093-691X [gefunden am 2005-01-15]

## Beschreibung

Die vorliegende Erfindung betrifft ein Verfahren zur funktionellen Beurteilung von Spermien.

Die schnelle und effiziente Beurteilung von Ejakulaten spielt sowohl in der Humanmedizin als auch in der Tiermedizin - insbesondere in der Nutztierzucht - eine wichtige Rolle. Bisher werden zur Beurteilung von Spermien und zur Prognose der Fertilität fast ausschließlich das Aussehen der Spermien (Morphologie) sowie die Beweglichkeit (Motilität) und die Vitalität (Durchlässigkeit der Membran am Spermienkopf für den Farbstoff Eosin) beurteilt. Wie die Praxis zeigt, sind aber diese Kriterien nicht ausreichend, um die Fertilität prognostisch zu beurteilen. Auch Menschen und Tiere, die hinsichtlich dieser Kriterien sehr gut abschneiden, haben schlechte Befruchtungsraten. Selbst die lange als äußerst wichtig eingeschätzte Motilität der Spermien erlaubt nur eine eingeschränkte Beurteilung, da die Spermien im weiblichen Genitale in erster Linie durch Kontraktionen der glatten Muskulatur transportiert werden. Die eigene Beweglichkeit der Spermien ist lediglich wichtig, um in die Mitte des Lumens des Eileiters zu gelangen, da am Rande des Eileiters starke Flüssigkeitsverwirbelungen durch die Aktivität der Kinozilien auftreten.

In Rinderbesamungsstationen wird die Qualität des Ejakulats eines Bullen beispielsweise vor und nach dessen Verdünnung und Abfüllung in Pailletten, vor der Kryokonservierung, nach der Kryokonservierung und vor dem Versenden in das In- und Ausland kontrolliert. Bisher werden als Parameter zur Beurteilung von Spermien deren Morphologie, Motilität (Prozentsatz der vorwärtsbeweglichen Spermien) und Vitalität untersucht. Diese Parameter sind zur Prognose der Fertilität eines Bullen jedoch leider nicht ausreichend. So stellt sich bei einigen Bullen im Durchschnitt erst nach drei Jahren, wenn bereits zahlreicher Besamungen durchgeführt wurden, heraus, dass sie sehr schlechte Befruchtungsergebnisse aufweisen, d.h. nur unzureichende Fertilität - und dies trotz einer hervorragenden Beurteilung der Spermien nach den bisher in der Routine verwendeten Kriterien Morphologie, Motilität und Vitalität. Der wirtschaftliche Schaden ist in diesen Fällen groß, da die Bullen erst drei Jahre aufgestallt werden, um im vierten Jahr das Sperma zu gewinnen. Zudem werden erstmals sechs bis neun Monate später die Ergebnisse des Besamungserfolgs statistisch ausgewertet. Ein "schlechter" Bulle schadet daher nicht nur dem Ruf der Besamungsstation - er stellt auch einen kompletten finanziellen Ausfall dar, da er bei Ausbleiben des gewünschten Befruchtungserfolges, also mangelnder Fertilität geschlachtet werden muss und die gesamten gewonnenen Samenportionen vernichtet werden. Aktuell ist kein Schnelltestsystem kommerziell erhältlich, der eine funktionelle Qualitätsbeurteilung hinsichtlich der Fertilität von bovinen Ejakulaten zuverlässig erlaubt.

In der Humanmedizin wurde von der Firma Origio ein Hyaluronsäure-Bindungstest entwickelt, der dazu dienen sollte, über die Bindungsfähigkeit an einen Tropfen Hyaluronsäure, gezielt gute Spermien für die intrazytoplasmatische Spermieninjektion zu selektieren. Mit dieser Methode konnten jedoch bisher keine signifikant besseren Befruchtungsergebnisse erzielt werden als mit den bisher eingesetzten Standardverfahren.

D. Waberski et al. offenbart in Binding of broar spermatozoa to oviductal ephitelium in vitro in relation to sperm morphology and storage time, Reproduction, Bd. 131, Nr. 2, 1. Februar 2006 (2006-02-01), Seiten 311-318, dass eine wichtige Eigenschaft im Hinblick auf die Funktionalität von Spermien ihre Fähigkeit ist, an Zellen im Eileiter zu binden. Es wird ein Bindungsassay an explantierte Epithelzellen des Eileiters verwendet um die Bindung von Spermien in verdünntem Samen von Ebern zu untersuchen. Die Anzahl der bindenden Spermien ist signifikant größer in Samenproben, die 24h gelagert wurden im Vergleich zur Lagerung über 72 Stunden. Im Gegensatz dazu sind andere, gängige Parameter zur Beurteilung der Spermienqualität, wie zum Beispiel Beweglichkeit, oder Färbung mit Propidiumoxid, nicht verändert. Die Autoren leiten daraus eine Verwendung eines Spermien-Eileiter Bindungstests zur Beurteilung der Spermien Funktion ab.

Ellington Joanna E. at al., Higher-quality human sperm in a sample selectively attach to oviduct (fallopian tube) epithelial cells in vitro, Fertility and Sterility, Bd. 71, Nr. 5, Mai 1999 (1999-05), Seiten 924-929 offenbart, dass Spermien, die nicht an Eileiter Epithelzellen binden, eine verringerte Mobilität, mehr Membrandefekte und mehr Akrosomreaktionen aufweisen als Kontrollspermien. Spermien, die an Epithelzellen binden, weisen weniger Defekte auf als solche, die das nicht tun. Selektive Bindung scheint wichtig zu sein um ein Reservoir an qualitativ hochwertigen Spermien im Eileiter zu binden. Es wird bereits auf die Möglichkeit zellfreier Assays hingeweisen.

Waberski D. et al., Importace of sperm-bindng assays for fertility prognosis of porcine spermatozoa, Theriogenology, Los Altos, Ca, US, Bd. 63, Nr. 2, 15. Januar 2005 (2005-01-15), Seiten 470-484 bezieht sich auf die Verwendung von Spermien-Eileiter Bindungstests zur Beurteilung der Qualität von Spermienzellen. Es wird ein Bindungstest mit explantierten Eileiter Epithelzellen beschrieben. Darüber hinaus werden die in unterschiedlichen Spezies für die Spermienbindung verantwortlichen Kohlenhydratstrukturen erwähnt.

WO2013/012749 bezieht sich auf Methoden zur Evaluieung der Bindung von Spermien an Oozyten und im Speziellen an Kohlenhydratstrukturen. Besagte Kohlenhydratstrukturen, die zum Beispiel Sialyl Lewis X Saccharide und Fucose enthalten, werden auf einer Matrix immobilisiert. Die Matrix kann zum Beispiel Plastik oder Agarose sein. Nach Bindung an die Matrix werden die Zellen visualisiert, gegebenenfalls verstärkt über eine immunohistochemische Anfärbung. Die gebundenen Zellen können mittels automatisierter Zählung oder Durchflusszytometrie quantifiziert werden.

Mithin ist ein Test, der eine prognostische Beurteilung der Fertilität von Spermieneines Bullensperma, ermöglicht, wünschenswert. Dieser Test sollte eine einfache, schnelle und doch sicherere Begutachtung erlauben, als die durch die konventionellen Tests möglich ist.

Revah, I. et al. offenbart in Biology of Reproduction, Bd. 62, Nr. 4, April 2000, Seiten 1010-1015 eine zellfreie Methode zur die Diktion der Bindung von Bullen Spermien an Fucose. Spermien werden mit fluoreszenzmarkierter Fucose in kopiert und die Bindung mittels FACS oder Epifluoreszenzmikroskopie analysiert. Die Autoren schließen, dass die Bindung von Spermien an Fucose für die Bildung eines Spermienreservoirs im Eileiter verantwortlich ist.

Suarez, SS, offenbart in einem Übersichtsartikel in Reproduction in Domestic Animals, Bd. 37, Nr. 3, 1. Juni 2002, Seiten 140-143 unter anderem die Relevanz der Bildung eines Spermienreservoirs im Eileiter für den Befruchtungsvorgang bei Nutztieren.

Rath, D. et al. berichtet in einem Übersichtsartikel in Reprod Dom Anim 43 (Suppl. 5), 2-11 (2008) über Spermien Interaktionen zwischen Insemination und Befruchtung. Sie Vorder ein besseres Verständnis zu entwickeln für die hochkomplexen Prozesse, die zwischen der Besamung und Befruchtung stattfinden, um einerseits die Effizienz konventioneller Reproduktionstechniken zu verbessern und andererseits die Entwicklung und Einführung neuer Reproduktionstechniken zu ermöglichen.

Das der Erfindung zugrunde liegende Problem wird durch ein Verfahren gelöst, das die ejakulatbezogene Fertilität von Rinderbullen mittels Untersuchung von Funktionen von in einem Ejakulat des Rinderbullen enthaltenen Spermien bestimmt und dadurch gekennzeichnet ist, dass als Funktion der in den Ejakulaten enthaltenen Spermien die Bindungsfähigkeit zumindest eines Anteils der Spermien aus dem Ejakulat an Fucose oder eine die Fucose enthaltende Verbindung in einem zellfreien Assay bestimmt wird, wobei eine gegenüber einer Kontrolle erhöhte Bindungsfähigkeit der Spermien an die Fucose oder eine die Fucose enthaltende Verbindung eine höhere Befruchtungsfähigkeit der Spermien und damit eine höhere Qualität des Ejakulats Fertilität des Rinderbullen anzeigt.

Unter einer Fucose enthaltenden Verbindung wird eine Substanz verstanden, die Fucose als Strukturelement aufweist. Dabei kann die Fucose beispielsweise an eine weitere Substanz chemisch gekoppelt vorliegen, wobei die Kopplung nicht zu einem Verlust der Bindungsfähigkeit der Fucose enthaltenden Verbindung an Spermien führen soll. In welcher Art Fucose an geeignete Substanzen gekoppelt werden kann, sei es durch Ionenbindung, Komplexbildung oder kovalente Bindung, ist dem Fachmann bekannt. Auch die zur Kopplung der Fucose geeigneten Substanzen sind dem Fachmann aufgrund seines Fachwissens geläufig.
Fig. 1: Zahl der gebundenen Spermien pro 0.02 mm² in Abhängigkeit von der NRR 90.
Fig. 2: Änderungen der Bindungsfähigkeit der Spermien eines Rinderbullen im Verlauf eines Jahres.
Fig. 3: Bindungsfähigkeit der Spermien in Abhängigkeit von dem verwendeten Verdünner.
Fig. 4: Einfluss verschiedener Verdünner auf die Bindungsfähigkeit der Spermien einzelner Rinderbullen.

In einer Ausführungsform des erfindungsgemäßen Verfahrens tritt die Fucose oder die Fucose enthaltende Verbindung in gelöster oder dispergierter Form mit den Spermien in Wechselwirkung.

In einer anderen Ausführungsform des erfindungsgemäßen Verfahrens ist die Fucose oder die Fucose enthaltende Verbindung auf einem Träger angeordnet.

Insbesondere kann erfindungsgemäß ein im Bereich des sichtbaren Lichts transparenter Träger zur Selektion bindungsfähiger Spermien verwendet werden. Als Alternative kommt erfindungsgemäß ein poröser Träger oder eine Säule zur spezifischen Selektion bindungsfähiger Spermien verwendet werden.

Erfindungsgemäß kann die Selektion bindungsfähiger Spermien mittels Zellsortierung, insbesondere FACS erfolgen.

Erfindungsgemäß kann ein durchlässig poröser Träger in Form einer transparenten Membran verwendet werden.

Erfindungsgemäß kann zur Selektion bindungsfähiger Spermien ein bildgebendes Verfahren, gegebenenfalls in Kombination mit colorimetrischen, fluorimetrischen oder luminometrischen Detektion verwendet erden. Auch eine nicht transparente Membran kann als Träger dienen und in Kombination mit einer colorimetrischer, fluorimetrischer oder luminometrischer Detektionsmethode verwendet werden.

Erfindungsgemäß kann der Bindungs-Assay zur Bewertung der Qualität von Spermaverdünnern herangezogen werden.

Im erfindungsgemäßen Verfahren kann ein glatter Träger verwendet werden und die Spermien können direkt durch Fluoreszenzmarkierung oder Anfärben mit einem Farbstoff (z.B. Färbung nach Richardson, Färbung mit Methylenblau) detektiert werden oder aber indirekt durch Anfärbung eines den Spermienkopf umgebenden Hofes.

Die Fucose oder Fucose enthaltende Verbindung kann dabei in einem gelatinösen Medium auf dem Träger vorliegt.

Fucose oder Lewisantigen können auch über eine kovalente Bindung auf einem Träger fixiert sein.

In einer anderen Ausführungsform des erfindungsgemäßen Verfahrens ist die eileiterspezifische Struktur auf einem Träger angeordnet. Dabei ist der Träger entweder durchlässig porös (3D) oder glatt (2D). Bei den durchlässig porösen Trägern kommen entweder Säulen, transparente Membranen oder nicht transparente Membranen zur Anwendung. Werden Säulen verwendet, wird eine Affinitätschromatographie durchgeführt. Bei nichttransparenten Membranen wird eine lumineszente Membran oder aber eine Fluoreszenzmarkierung der Spermien zur Detektion verwendet.

Bei den glatten Trägern werden die Spermien durch Fluoreszenzmarkierung oder aber durch Anfärbung mit spezifischen Farbstoffen detektiert. Es besteht auch die Möglichkeit der indirekten Spermiendetektion durch Anfärbung eines Hofes um den Spermienkopf.

In einer weiteren Ausführungsform des erfindungsgemäßen Verfahrens werden die die Bindung des Spermiums vermittelnden Kohlehydrate in gelatinösem Medium gelöst.

Die Erfindung umfasst ein kommerzielles Schnelltestsystem zur funktionellen Qualitätsbeurteilung von Ejakulaten und zur Prognose der Fertilität.

Die Erfindung basiert auf der Entdeckung, dass die Bindungsfähigkeit der Spermien im Eileiter mit der Befruchtungsfähigkeit korreliert ist.

Der Vorteil der Erfindung liegt darin, dass ein darauf beruhender -Bindungs-Assay einen Schnelltest zur funktionellen Analyse der Spermienqualität zur Verfügung stellt. Dies war bisher nicht möglich und daher in den Routineuntersuchungen weder in der Humanmedizin noch in der Tiermedizin vorgesehen.

Mit dem erfindungsgemäßen Verfahren, insbesondere seiner Eignung als Schnelltest, wird es erstmals ermöglicht, frühzeitig und gezielt eine prognostische Aussage über die Fertilität zu erstellen.

Die Erfinder haben ein neues digitales videomikroskopischen Analysesystems entwickelt, das es erstmals ermöglicht, die Vorgänge des Eizell- und Spermientransports, der Befruchtung und der frühen Embryonalentwicklung im Eileiter unter nahen *in vivo* Bedingungen zu untersuchen. Spermien binden - sobald sie den Eileiter erreichen - an die Eileiterepithelzellen und bilden ein sogenanntes Spermienreservoir aus. Auf diese Weise bleiben sie drei bis vier Tage vital und befruchtungsfähig. Diese Bindung wird durch spezielle Lektine am Kopf des Spermiums und einem an den Kinozilien befindlichen Zucker vermittelt. Beim Rind handelt es sich beispielsweise um die Lektine BSP A/B, bei dem an den Kinozilien an der Bindung beteiligten Zucker handelt es sich um Fucose (Suarez und Kölle, 1989). Mit Hilfe des neuen videomikroskopischen Analysesystems konnten die Erfinder unter nahen *in vivo* Bedingungen erstmals zeigen, dass die Bindungsfähigkeit der Spermien im Eileiter mit der Fertilität korreliert ist. So sind bei Rinderbullen mit einer Non-Return-Rate 90 (NRR90, Prozentsatz der Kühe, die 90 Tage nach der Besamung trächtig sind) > 55 % signifikant mehr Spermien am Eileiterepithel gebunden als bei Rinderbullen mit einer NRR90 <45 % (T-Test, p<0,01).

Zudem konnten die Erfinder zeigen, dass bei Rinderbullen geringer Fertilität ein signifikant höherer Prozentsatz der gebundenen Spermien nicht mehr motil ist (T-Test, p<0.001). Fehlende Motilität hat zur Folge, dass bei diesen Spermien keine Hyperaktivierung erfolgt. Dies bedeutet, dass diese Spermien sich nicht mehr vom Eileiterepithel losreißen können, sobald sich eine befruchtungsfähige Eizelle im Ovidukt befindet. Damit können die Spermien nicht mehr erfolgreich zur Eizelle wandern. Zudem konnten die Erfinder erstmals nachweisen, dass die Qualität eines Ejakulats nicht konstant ist, sondern deutlich durch den Gesundheitszustand beeinflusst wird. Jede Störungen des Allgemeinbefindens, z. B. infolge einer Grippe oder einer Gelenkverletzung, führt umgehend zu einer Verringerung der Spermienbindung im Eileiter und zu einer verminderten Fertilität. Die Sensitivität der Spermienbindung ist so hoch, dass selbst bei einer subklinischen Erkrankung, bei der noch keine deutlichen klinischen Symptome auftreten, die Bindungskapazität der Spermien vermindert ist. Auch sehr hohe Temperaturen oder eine hohe Luftfeuchtigkeit führen zu einer deutlichen Verminderung der Spermienqualität.

Da natives Eileiterepithel (beispielsweise aus Kühen unmittelbar nach der Schlachtung) in Besamungsstationen nicht verfügbar ist und zudem ein Kontaminationsrisiko birgt, entwickelten die Erfinder - basierend auf den oben geschilderten Entdeckungen - die technische Umsetzung: einen Spermien-Bindungs-Assay auf einem Objektträger, der die Bindung der Spermien am Eileiterepithel imitiert. Zu diesem Zweck werden Objektträger mit einer speziellen Matrix, die Gelatine und Fucose enthält, beschichtet. Die zu untersuchenden Spermien werden auf dem Objektträger bei 37 Grad inkubiert. In anschließenden Waschschritten werden die nicht gebundenen Spermien entfernt, Die auf dem Objektträger gebundenen Spermien werden nach Richardsen gefärbt und mit Hilfe eines Computerprogramms (Image Pro) pro Flächeneinheit ausgezählt. Bei der Untersuchung von Sperma von mehr als 100 Rinderbullen konnten die Erfinder zeigen, dass die Zahl der gebundenen Spermien mit der Befruchtungsfähigkeit der Spermien korreliert ist und damit zu einer prognostischen Beurteilung der Fertilität eines Rinderbullen geeignet ist.

### Ausführungsbeispiele

### Ausführungsbeispiel 1

Untersuchung von 40 Ejakulaten von Testbullen hoher (NRR 90>47 %) mittlerer (NRR 90 >42%, <47 %) und niedriger Fertilität (NRR 90 <42%)
a) Beschichtung der Objektträger mit 600 mM Fucose
   - 0,23 g Gelatine auf 50 ml Aqua dest. auffüllen, unterrühren und auf 75 °C erhitzen -> Gelatine löst sich
   - Abkühlen lassen auf 50 °C
   - 2 ml Chromalaunlösung zugeben (0,8 g Chromalaun auf 20 ml Aqua dest.)
   - davon 15 ml zu 1,44 g Fucose geben
   - Lösung 5 min. mit Rührstäbchen durchmischen
   - Objektträger (OT) bei 50 °C 5 min. beschichten; dazu Objektträger in eine Küvette mit einem Rührstäbchen auf Heizplatte stellen
b) Auftauen der Spermien
   - Spermienpailette in flüssigen Stickstoff lagern und transportieren.
   - 0,25 ml Paillette 10-30 sec Sekunden im Wasserbad bei 38 Grad auftauen und gut mit Tuch von Wasser befreien (Spermien werden durch Wasser geschädigt.).
   - Auf der einen Seite ohne Stopfen mit einer Schere aufschneiden und mit der offenen Seite nach unten in ein handwarmes Eppendorf-Gefäß stellen.
   - Jetzt auch die 2. Seite aufschneiden und Ejakulat in ein Eppendorf-Gefäß geben (0,25 oder 0,5 ml/Paillette: eine Paillette von 0,25 ml enthält 15 Mio Spermien)
   - Es bleibt ein Rest in der Paillette zurück, ca. 10µl,, welcher auf einen Objektträger aufgetragen wird. Beurteilung von Morphologie und Motilität.
c) Waschen der Spermien
   - Am Vortag incomplete TALP ansetzen/ auftauen, steril filtrieren und über Nacht im offenen Gefäß im Brutschrank bei 38° C und 5% CO2 äquilibrieren.
   - ca. 500 - 1000µl incomplete TALP zur Spermiensuspension zugeben und
      gut vermischen.
   - Zentrifugieren bei ∼1200g, 5 Minuten.
   - Überstand über Pellet mit Pipette vorsichtig entfernen.
   - Nochmals ca. 500 - 1000µl incomplete TALP zur Spermiensuspension zugeben und gut mischen
   - Zentrifugieren bei ∼1200g 5 Minuten.
   - Überstand über Pellet vorsichtig mit Pipette abnehmen.
   - Pellet in 100 µl incomplete TALP lösen, Motilität und Vitalität am Phasenkontrastmikroskop überprüfen
d) Verdünnen der Spermien
   - 1 µl dieser Lösung mit 19 µl Aqua bidest mischen und mittels Neubauer-Zählkammer auszählen
   - Verdünnung auf 100000 Spermien/ µl
e) Durchführung des Bindungs-Assays
   - 7 µl des verdünnten Ejakulats auf spezialbeschichteten Objektträger aufbringen
   - 7 min bei Raumtemperatur inkubieren
   - 3xWaschen in Küvette mit iTALP
   - auf 38 °C warmer Heizplatte trocknen
   - mit Methanol (unter dem Abzug) in Standküvette 7 min fixieren
   - auf Heizplatte bei70 °C 20 s mit Richardson färben
   - 3xWaschen in Aqua dest.
   - auf der Heizplatte bei 70 °C trocknen lassen.
   - mit Epon eindeckeln, über Nacht trocknen lassen.
   - Auszählen von Spermien in 4 verschiedenen Gesichtsfeldern (300-fache Vergrößerung) mit Hilfe der Software Image J Pro.

### Ergebnis:

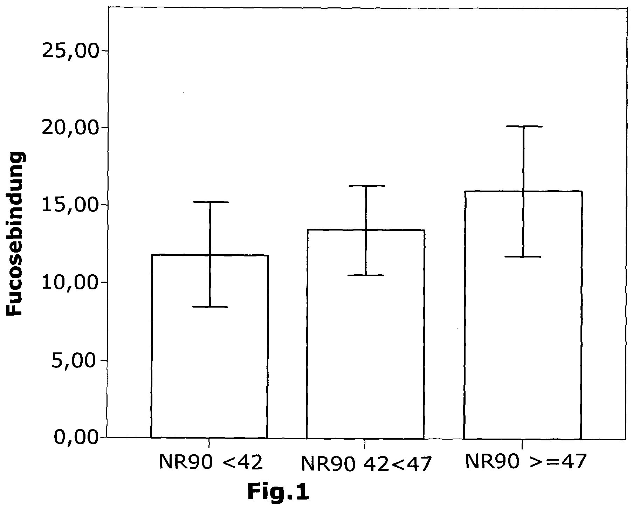

Bei Bullen mit einer geringen Fertilität sind weniger Spermien gebunden als bei Bullen hoher Fertilität (Fig. 1).

Fig. 1: Zahl der gebundenen Spermien pro 0.02 mm² in Abhängigkeit von der NRR 90. Bei Bullen geringer Fertilität sind weniger Spermien gebunden als bei Bullen hoher Fertilität (Kruskal-Wallis-Test, p<0.02).

Nach Durchführung des Bindungs-Assays bei 30 hoch fertilen, 30 mäßig fertilen und 25 subfertilen/infertilen Bullen wurde die Auswertung des Tests erarbeitet: Sind weniger als 150 Spermien pro 0,04 mm² vorhanden, ist der Bulle subfertil/infertil. 150-250 Spermien pro 0,04 mm² sprechen für mäßige Fertilität. Mehr als 250 gebundene Spermien pro 0,04 mm² lassen auf hohe Fertilität schließen. Diese Zahlenwerte gelten für die Milchrinderrasse Holstein-Friesian.

Unsere Versuche an verschiedenen Rinderrassen haben gezeigt, dass die Auswertungsgrenzen der Anzahl der gebundenen Spermien speziesspezifisch sind und für jede Rasse gesondert festgelegt werden müssen.

### Ausführungsbeispiel 2

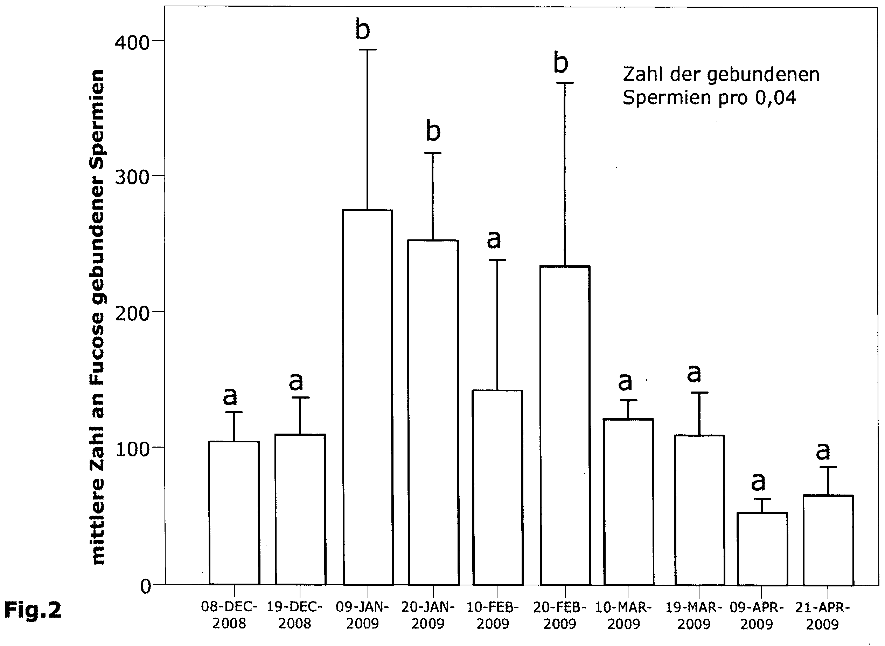

In diesem Experiment wurde das Ejakulat einen als hoch fertil eingestuften Bullen alle 2 Wochen mit Hilfe des Bindungs-Assays analysiert. Die Versuchsdurchführung erfolgte wie oben beschrieben. Es zeigte sich, dass die Qualität des Ejakulats nicht konstant ist. Vielmehr zeigte sich, dass die Bindungsfähigkeit der Spermien in Abhängigkeit von Klima und Gesundheitszustand schwankt. So war z. B. die Bindungsfähigkeit im Januar 2009 sehr gut - in diesem Monat gab es bei einer Durchschnittstemperatur von 0,2 Grad lediglich 7mm Niederschlag. Im April 2009, in dem bei einer Durchschnittstemperatur von 11,8 Grad 50 mm Niederschlag fiel und eine sehr hohe Luftfeuchtigkeit im Stall herrschte, sank die Bindungsfähigkeit signifikant (Fig. 2, Anova, p<0,05)

Fig. 2: Änderungen der Bindungsfähigkeit der Spermien eines Bullen im Verlauf eines Jahres (Anova, p<0,05).

### Ausführungsbeispiel 3

In einem weiteren Experiment wurde die Bindungsfähigkeit der Spermien im Bindungs-Assay in Abhängigkeit von dem verwendeten Verdünner untersucht. Dazu wurden die Ejakulate von 4 verschiedenen Bullen in jeweils 3 verschiedenen Verdünnern verwendet. Die experimentelle Durchführung erfolge wie unter Ausführungsbeispiel 1 beschrieben. Allerdings musste bei den Spermien in eigelbhaltigen Verdünner jeweils 1 zusätzlicher Waschschritt eingefügt werden, um die Proteine des Eigelbs zu entfernen.

Die Untersuchungen zeigten, dass Spermien im eigelbhaltige Verdünner 3 eine signifikant erhöhte Bindungsfähigkeit zeigen (p<0,05, Mann-Whitney Test).

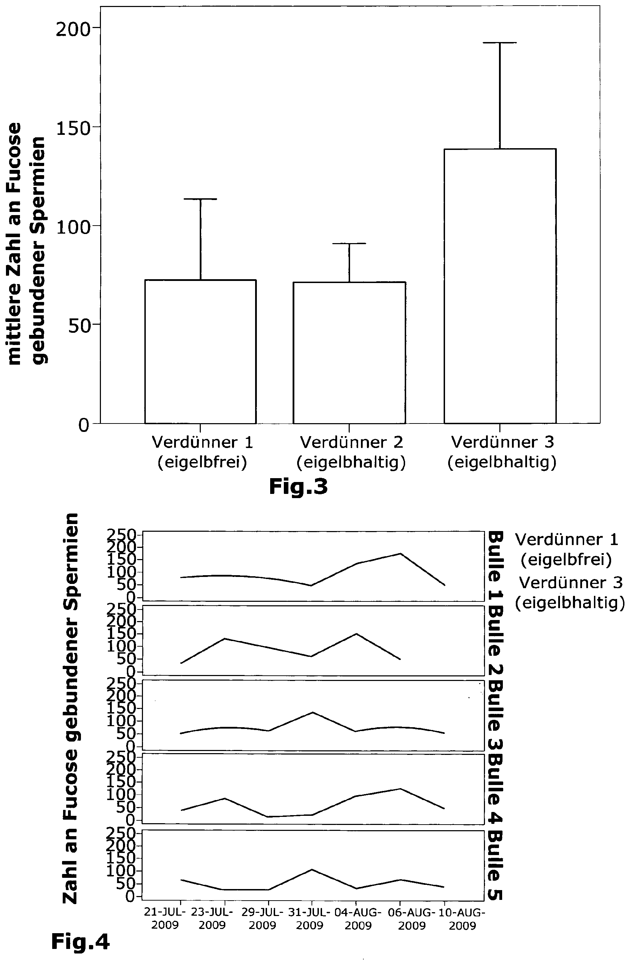

Fig. 3: Bindungsfähigkeit der Spermien in Abhängigkeit von dem verwendeten Verdünner. Verdünner 3 erhöht signifikant die Bindungsfähigkeit der Spermien(p<0,05, Mann-Whitney-Test).

### Ausführungsbeispiel 4

In diesem Experiment wurde die Bindungsfähigkeit von Spermien verschiedener Bullen mit verschiedenen Verdünnern getestet. Die Durchführung der Versuche erfolgte analog zu Ausführungsbeispiel 3. Es stellte sich heraus, dass die Verdünner die Bindungsfähigkeit von Spermien individuell unterschiedlich modulieren. So kann Verdünner 3 bei Bulle 5 eine Verbesserung der Bindungsfähigkeit bewirken, während er bei Bulle 2 kaum Verbesserungen bewirken kann (Fig. 3).

Fig. 4: Einfluss verschiedener Verdünner auf die Bindungsfähigkeit der Spermien einzelner Bullen. Der Einfluss des Verdünners auf die Bindungskapazität der Spermien ist individuell unterschiedlich.

## Patentansprüche

1. Verfahren zur funktionellen, ejakulatsbezogenen Qualitätsbeurteilung der Spermien eines Rinderbullen zur Prognose der Fertilität mittels Untersuchung von Funktionen von in einem Ejakulat des Rinderbullen enthaltenen Spermien **dadurch gekennzeichnet, dass** als Funktion der in den Ejakulaten enthaltenen Spermien die Bindungsfähigkeit zumindest eines Anteils der Spermien aus dem Ejakulat an Fucose oder eine die Fucose enthaltende Verbindung in einem zellfreien Assay bestimmt wird, wobei eine gegenüber einer Kontrolle erhöhte Bindungsfähigkeit der Spermien an die Fucose oder eine Fucose enthaltende Verbindung eine höhere Befruchtungsfähigkeit der Spermien und damit eine höhere Qualität des Ejakulats anzeigt.

2. Verfahren nach Anspruch 1, **dadurch gekennzeichnet, dass** die Fucose oder eine Fucose enthaltende Verbindung in gelöster oder dispergierter Form mit den Spermien in Wechselwirkung tritt.

3. Verfahren nach Anspruch 1 oder 2, **dadurch gekennzeichnet, dass** die Fucose oder eine Fucose enthaltende Verbindung auf einem Träger angeordnet ist.

4. Verfahren nach Anspruch 3, **dadurch gekennzeichnet, dass** ein im Bereich des sichtbaren Lichts transparenter Träger zur Selektion bindungsfähiger Spermien verwendet wird.

5. Verfahren nach 3 oder 4, **dadurch gekennzeichnet, dass** ein poröser Träger zur Selektion bindungsfähiger Spermien verwendet wird.

6. Verfahren nach mindestens einem der Ansprüche 1 bis 3, **dadurch gekennzeichnet, dass** spezifische Säulen zur Selektion bindungsfähiger Spermien verwendet werden.

7. Verfahren nach mindestens einem der Ansprüche 1 bis 6, **dadurch gekennzeichnet, dass** die Selektion bindungsfähiger Spermien mittels Zellsortierung, insbesondere FACS erfolgt.

8. Verfahren nach mindestens einem der Ansprüche 1 bis 7, **dadurch gekennzeichnet, dass** eine Selektion bindungsfähiger Spermien mittels Bindung der Spermien an Säulen erfolgt.

9. Verfahren nach mindestens einem der Ansprüche 3 bis 5, **dadurch gekennzeichnet, dass** ein durchlässig poröser Träger in Form einer transparenten Membran verwendet wird.

10. Verfahren nach Anspruch 9, **dadurch gekennzeichnet, dass** zur Selektion bindungsfähiger Spermien ein bildgebendes Verfahren gegebenenfalls in Kombination mit colorimetrischen, fluorimetrischen oder luminometrischen Detektion verwendet wird.

11. Verfahren nach mindestens einem der Ansprüche 3 und 5-8 **dadurch gekennzeichnet, dass** eine nicht transparente Membran als Träger dient und in Kombination mit einer colorimetrischen, fluorimetrischen oder luminometrischen Detektionsmethode verwendet wird.

12. Verfahren nach mindestens einem der Ansprüche 1 bis 11, **dadurch gekennzeichnet, dass** ein Bindungs-Assay zur Bewertung der Qualität von Spermaverdünnern verwendet wird.

13. Verfahren nach mindestens einem der Ansprüche 3 bis 12, **dadurch gekennzeichnet, dass** ein glatter Träger verwendet wird und die Spermien direkt durch Fluoreszenzmarkierung oder Anfärben mit einem Farbstoff (z.B. Färbung nach Richardson, Färbung mit Methylenblau) detektiert werden oder aber indirekt durch Anfärbung eines den Spermienkopf umgebenden Hofes.

14. Verfahren nach mindestens einem der Ansprüche 3 bis 13, **dadurch gekennzeichnet, dass** Fucose in einem gelatinösen Medium auf dem Träger vorliegt.

15. Verfahren nach mindestens einem der Ansprüche 3 bis 13, **dadurch gekennzeichnet, dass** Fucose oder Lewisantigen über eine kovalente Bindung auf einem Träger fixiert sind.

## Claims

1. A process for the functional ejaculate-related quality evaluation of the spermatozoa of a male bovine for prognosticating the fertility by examining functions of spermatozoa contained in an ejaculate of said male bovine, **characterized in that** the binding capacity of at least a fraction of the spermatozoa from the ejaculate to fucose or a compound containing fucose is determined as a function of the spermatozoa contained in the ejaculate in a cell-free assay, wherein a binding capacity of the spermatozoa to the fucose or to a compound containing fucose that is increased over that of a control indicates a higher fertility of the spermatozoa and thus a higher quality of the ejaculate.

2. The process according to claim 1, **characterized in that** said fucose or a compound containing fucose interacts with the spermatozoa in a dissolved or dispersed form.

3. The process according to claim 1 or 2, **characterized in that** said fucose or a compound containing fucose is arranged on a support.

4. The process according to claim 3, **characterized in that** a support that is transparent in the range of visible light is used for the selection of spermatozoa having binding capacity.

5. The process according to claim 3 or 4, **characterized in that** a porous support is used for the selection of spermatozoa having binding capacity.

6. The process according to at least one of claims 1 to 3, **characterized in that** specific columns are used for the selection of spermatozoa having binding capacity.

7. The process according to at least one of claims 1 to 6, **characterized in that** the selection of spermatozoa having binding capacity is effected by means of cell sorting, especially FACS.

8. The process according to at least one of claims 1 to 7, **characterized in that** a selection of spermatozoa having binding capacity is effected by binding the spermatozoa to columns.

9. The process according to at least one of claims 3 to 5, **characterized in that** a permeable porous support in the form of a transparent membrane is used.

10. The process according to claim 9, **characterized in that** an imaging method, optionally in combination with colorimetric, fluorimetric or luminometric detection, is used for the selection of spermatozoa having binding capacity.

11. The process according to at least one of claims 3 and 5 to 8, **characterized in that** a non-transparent membrane serves as the support and is used in combination with a colorimetric, fluorimetric or luminometric detection method.

12. The process according to at least one of claims 1 to 11, **characterized in that** a binding assay is used for evaluating the quality of sperm thinners.

13. The process according to at least one of claims 3 to 12, **characterized in that** a smooth support is used and the spermatozoa are detected directly by fluorescence labeling or staining with a dye (e.g., staining according to Richardson, staining with methylene blue), or indirectly by staining a halo surrounding the sperm head.

14. The process according to at least one of claims 3 to 13, **characterized in that** fucose is present on the support in a gelatinous medium.

15. The process according to at least one of claims 3 to 13, **characterized in that** fucose or Lewis antigen is fixed on a support through a covalent bond.

## Revendications

1. Procédé destiné à l'évaluation de la qualité fonctionnelle des spermatozoïdes d'un taureau sur la base de son éjaculat en vue de diagnostiquer sa fertilité au moyen de l'analyse des fonctions des spermatozoïdes contenus dans un éjaculat du taureau, **caractérisé en ce que** la capacité de liaison au fucose, ou à une liaison contenant du fucose, de tout au moins une partie des spermatozoïdes est déterminée en tant que fonction des spermatozoïdes contenus dans les éjaculats, à partir de l'éjaculat dans une analyse acellulaire, dans laquelle une capacité de liaison des spermatozoïdes au fucose ou à une liaison contenant du fucose, est accrue par rapport à un examen, et indique une capacité de fécondation plus élevée des spermatozoïdes et par conséquent une qualité plus élevée de l'éjaculat.

2. Procédé selon la revendication 1, **caractérisé en ce que** le fucose ou une liaison contenant du fucose apparaît en alternance avec les spermatozoïdes sous une forme dissoute ou dispersée.

3. Procédé selon la revendication 1 ou 2, **caractérisé en ce que** le fucose ou une liaison contenant du fucose est disposé(e) sur un support.

4. Procédé selon la revendication 3, **caractérisé en ce qu'**un support transparent à la lumière visible est employé en vue de la sélection des spermatozoïdes aptes à être liés.

5. Procédé selon la revendication 3 ou 4, **caractérisé en ce qu'**un support poreux est employé en vue de la sélection des spermatozoïdes aptes à être liés.

6. Procédé selon au moins l'une des revendications 1 à 3, **caractérisé en ce que** des colonnes spécifiques sont employées en vue de la sélection des spermatozoïdes aptes à être liés.

7. Procédé selon au moins l'une des revendications 1 à 6, **caractérisé en ce que** la sélection des spermatozoïdes aptes à être liés a lieu au moyen d'un tri cellulaire, en particulier au moyen d'une opération de tri cellulaire par cytofluorométrie (FACS).

8. Procédé selon au moins l'une des revendications 1 à 7, **caractérisé en ce qu'**une sélection des spermatozoïdes aptes à être liés a lieu au moyen de la liaison des spermatozoïdes à des colonnes.

9. Procédé selon au moins l'une des revendications 3 à 5, **caractérisé en ce qu'**un support poreux et de nature perméable est employé sous la forme d'une membrane transparente.

10. Procédé selon la revendication 9, **caractérisé en ce que** qu'une méthode par imagerie est employée en vue de la sélection des spermatozoïdes aptes à être liés, le cas échéant en combinaison avec une méthode de détection colorimétrique, fluorimétrique ou luminométrique.

11. Procédé selon au moins l'une des revendications 3 et 5 à 8, **caractérisé en ce qu'**une membrane non transparente sert de support et est employée en combinaison avec une méthode de détection colorimétrique, fluorimétrique ou luminométrique.

12. Procédé selon au moins l'une des revendications 1 à 11, **caractérisé en ce qu'**une analyse de la liaison est employée en vue de l'appréciation de la qualité des diluants de sperme.

13. Procédé selon au moins l'une des revendications 3 à 12, **caractérisé en ce qu'**un support lisse est employé et **en ce que** les spermatozoïdes sont détectés directement par l'intermédiaire d'un marqueur fluorescent ou par la coloration au moyen d'un colorant (par exemple : coloration selon la méthode de Richardson, coloration avec du bleu de méthylène) ou aussi de manière indirecte par l'intermédiaire de la coloration d'une auréole entourant la tête des spermatozoïdes.

14. Procédé selon au moins l'une des revendications 3 à 13, **caractérisé en ce que** du fucose est présent sur le support, dans un milieu gélatineux.

15. Procédé selon au moins l'une des revendications 3 à 13, **caractérisé en ce que** du fucose ou des antigènes de Lewis sont fixés à un support par l'intermédiaire d'une liaison covalente.
